# EUROPEAN PATENT SPECIFICATION

(11) **EP 1 458 368 B1**
(45) Date of publication and mention of the grant of the patent: **21.11.2007**
(21) Application number: 02789574.7
(22) Date of filing: 27.11.2002
(51) Int. Cl.: A61K 31/00, A61K 31/138, A61P 25/28

(54) **USE OF NOREPINEPHRINE REUPTAKE INHIBITORS FOR THE TREATMENT OF COGNITIVE FAILURE**
VERWENDUNG VON NOREPINEPHRIN WIEDERAUFNAHMEHEMMERN ZUR BEHANDLUNG VON KOGNITIVEN STÖRUNGEN
UTILISATION D'INHIBITEURS DE RECAPTAGE DE LA NORADRENALINE DANS LE TRAITEMENT DE LA DEFAILLANCE COGNITIVE

(30) Priority: 11.12.2001 US 339174 P
(43) Date of publication of application: 22.09.2004
(73) Proprietor: ELI LILLY AND COMPANY, Indianapolis, Indiana 46285 (US)
(72) Inventor: BYMASTER, Franklin, Porter, Brownsburg, IN 46112 (US); GEHLERT, Donald, Richard, Indianapolis, IN 46202 (US); MCKINZIE, David, Lee, Whitestown, IN 46075 (US); YANG, Charles, Renkin, Indianapolis, IN 46220 (US)
(74) Representative: Hiscock, Ian James
(86) International application number: PCT/US2002/036132
(87) International publication number: WO 2003/049724

(56) References cited:
- EP-A- 0 534 756
- EP-A- 0 721 777
- WO-A-01/01973
- WO-A-01/27068
- WO-A-02/053104
- WO-A-02/053140
- FERGUSON J M ET AL: "Effects of reboxetine on Hamilton Depression Rating Scale factors from randomized, placebo-controlled trials in major depression." INTERNATIONAL CLINICAL PSYCHOPHARMACOLOGY, vol. 17, no. 2, March 2002 (2002-03), pages 45-51, XP009008693 March, 2002 ISSN: 0268-1315
- GEHLERT D R ET AL: "(R)-THIONISOXETINE, A POTENT AND SELECTIVE INHIBITOR OF CENTRAL ANDPERIPHERAL NOREPHINEPHRINE UPTAKE" LIFE SCIENCES, PERGAMON PRESS, OXFORD, GB, vol. 56, no. 22, 1995, pages 1915-1920, XP000602398 ISSN: 0024-3205

## Description

The invention belongs to the fields of pharmaceutical chemistry and central nervous system medicine, and provides a use for the treatment of cognitive failure.

Cognitive failure is the dysfunction or loss of cognitive functions, the processes by which knowledge is acquired, retained, and used. An estimated 2% of all Americans have some form and degree of cognitive failure, with about 15% of those over the age of 65 affected. Cognitive failure is most commonly due to delirium or dementia, but it may also occur in association with a number of other disorders.

A delerium is characterized by a disturbance of consciousness and a change in cognition that develop over a short period of time. Dementia is a chronic deterioration of intellectual function and other cognitive skills severe enough to interfere with the ability to perform activities of daily living. Although dementia may occur at any age, it primarily affects the elderly, presenting in more than 15% of persons over 65 years of age and in as many as 40% of persons over 80 years old. Dementia accounts for more than half of nursing home admissions. Dementia due to Alzheimer's disease affects four million Americans, at an annual cost of about $90 billion, including medical and nursing home care, social services, lost productivity, and early death. Alzheimer's disease accounts for more than 65% of the dementias in the elderly.

Non-Alzheimer's dementias include Lewy body dementia, vascular dementia, and Binswanger's dementia (subcortical arteriosclerotic encephalopathy). Dementia may also appear in patients with Parkinson's disease, progressive supranuclear palsy, Huntington's disease (chorea), Pick's disease, frontal lobe dementia syndromes, dementia pugilistica, normal-pressure hydrocephalus, subdural hematoma, Creutzfeldt-Jakob disease, Gerstmann-Sträussler-Scheinker disease, general paresis, AIDS, and schizophrenia.

Current treatments for cognitive failure include compounds that enhance cholinergic transmission such as donepezil, rivastigmine, and tacrine. The use of these agents is limited by side effects including changes in vision or balance, diarrhea, dizziness, fainting spells or falls, increase in frequency of passing urine or incontinence, nervousness, agitation, increased confusion, skin rash or hives, slow heartbeat or palpitations, stomach pain, sweating, uncontrollable movements, unusual bleeding or bruising, red or purple spots on the skin, vomiting, and weight loss. Another therapy is the administration of the ergot hydergine. Hydergine therapy may require six months to determine whether the drug has been effective, and side effects include nausea.

WO 01101973 discloses the use of reboxetine for the treatment of cognitive disorders due to general medical conditions.

Additional therapies are needed for the treatment of cognitive failure that are more efficacious and better tolerated than treatments that are currently available.

The present invention provides the use of a selective norepinephrine reuptake inhibitor selected from the group consisting of atomoxetine and (R)-N-methyl-3-(2-methylthiophenoxy)-3-phenylpropylamine for the preparation of a medicament for the treatment or prevention of cognitive failure.

The term "cognitive failure", as used herein, refers to the spectrum of cognitive dysfunctions ranging from mild cognitive impairment to deterioration of intellectual function and other cognitive skills severe enough to interfere with the ability to perform activities of daily living.

Many compounds, including those discussed at length below, are selective norepinephrine reuptake inhibitors, and no doubt many more will be identified in the future. In the practice of the present invention, it is intended to include reuptake inhibitors which show 50% effective concentrations of about 1000 nM or less, in the protocol described by Wong et al., Drug Development Research, 6, 397 (1985). The norepinephrine reuptake inhibitors useful for the present invention are characterized in being selective for the inhibition of neurotransmitter reuptake relative to their ability to act as direct agonists or antagonists at other receptors. It is preferred that the compounds useful for the present invention are selective for the inhibition of norepinephrine reuptake relative to direct agonist or antagonist activity at other receptors by a factor of at least ten. Preferably, compounds useful for the present invention are selective for the inhibition of norepinephrine reuptake relative to direct agonist or antagonist activity at other receptors by a factor of at least one hundred. Norepinephrine reuptake inhibitors useful for the present invention include:
Atomoxetine (formerly known as tomoxetine), (R)-(-)-N-methyl-3-(2-methylphenoxy)-3-phenylpropylamine, is usually administered as the hydrochloride salt. Atomoxetine was first disclosed in U.S. Patent #4,314,081. The word "atomoxetine" will be used here to refer to any acid addition salt or the free base of the molecule. See, for example, Gehlert, et al., Neuroscience Letters, 157, 203-206 (1993), for a discussion of atomoxetine's activity as a norepinephrine reuptake inhibitor; and
(R)-N-methyl-3-(2-methylthiophenoxy)-3-phenylpropylamine or a pharmaceutically acceptable salt thereof. (R)-N-methyl-3-(2-methylthiophenoxy)-3-phenylpropylamine is described in U.S. Patent 5,281,624, of Gehlert, Robertson, and Wong, and in Gehlert, et al., Life Sciences, 55 (22), 1915-1920, (1995). (R)-N-methyl-3-(2-methylthiophenoxy)-3-phenylpropylamine is there taught to be an inhibitor of norepinephrine reuptake in the brain.

While all compounds selected from the group consisting of atomoxetine and (R)-N-methyl-3-(2-methylthiophenoxy)-3-phenylpropylamine are useful, certain are preferred. The use of atomoxetine hydrochloride for the present invention is the most preferred embodiment of the present invention.

The dosages of the drugs used in the present invention must, in the final analysis, be set by the physician in charge of the case using knowledge of the drugs, the properties of the drugs in combination as determined in clinical trials, and the characteristics of the patient including diseases other than that for which the physician is treating the patient. General outlines of the dosages, and some preferred dosages, can and will be provided here.

Atomoxetine: In adults and older adolescents: from about 5 mg/day to about 200 mg/day; preferably in the range from about 60 to about 150 mg/day; more preferably from about 60 to about 130 mg/day; and still more preferably from about 50 to about 120 mg/day; in children and younger adolescents: from about 0.2 to about 3.0 mg/kg/day; preferably in the range from about 0.5 to about 1.8 mg/kg/day;
(R)-N-methyl-3-(2-methylthiophenoxy)-3-phenylpropylamine: from about 0.01 mg/kg to about 20 mg/kg; preferred daily doses will be from about 0.05 mg/kg to 10 mg/kg; ideally from about 0.1 mg/kg to about 5 mg/kg;

Cognitive failure presents in patients suffering from a number of other disorders. The present invention includes the use of a norepinephrine reuptake inhibitor selected from the group consisting of atomoxetine and (R)-N-methyl-3-(2-methylthiophenoxy)-3-phenylpropylamine for the manufacture of a medicament for treating cognitive failure presenting alone or where cognitive failure is associated with another disorder. Schizophrenic patients, for example, commonly exhibit symptoms that include cognitive failure. An embodiment of the present invention, therefore, is the use of a norepinephrine reuptake inhibitor selected from the group consisting of atomoxetine and (R)-N-methyl-3-(2-methylthiophenoxy)-3-phenylpropylamine for the manufacture of a medicament for treating cognitive failure associated with schizophrenia. Patients suffering from schizophrenia also frequently exhibit negative symptoms such as flat affect, asociality, anergia, avolition, and anhedonia. A further embodiment of the present invention is the use of a norepinephrine reuptake inhibitor selected from the group consisting of atomoxetine and (R)-N-methyl-3-(2-methylthiophenoxy)-3-phenylpropylamine for the manufacture of a medicament for treating the negative symptoms of schizophrenia.

It will be understood by the skilled reader that most or all of the compounds used in the present invention are capable of forming salts, and that the salt forms of pharmaceuticals are commonly used, often because they are more readily crystallized and purified than are the free bases. In all cases, the use of the pharmaceuticals described above as salts is contemplated in the description herein, and often is preferred, and the pharmaceutically acceptable salts of all of the compounds are included in the names of them. Especially preferred pharmaceutically acceptable salts are those formed with hydrochloric acid.

The pharmaceutical compositions are prepared in a manner well known in the pharmaceutical art. The carrier or excipient may be a solid, semi-solid, or liquid material that can serve as a vehicle or medium for the active ingredient. Suitable carriers or excipients are well known in the art. The pharmaceutical composition may be adapted for oral, inhalation, parenteral, or topical use and may be administered to the patient in the form of tablets, capsules, aerosols, inhalants, suppositories, solutions, suspensions, or the like.

The compounds useful for the present invention may be administered orally, for example, with an inert diluent or capsules or compressed into tablets. For the purpose of oral therapeutic administration, the compounds may be incorporated with excipients and used in the form of tablets, troches, capsules, elixirs, suspensions, syrups, wafers, chewing gums and the like. These preparations should contain at least 4% of the compound of the present invention, the active ingredient, but may be varied depending upon the particular form and may conveniently be between 4% to about 70% of the weight of the unit. The amount of the compound present in compositions is such that a suitable dosage will be obtained. Preferred compositions and preparations useful for the present invention may be determined by a person skilled in the art.

The tablets, pills, capsules, troches, and the like may also contain one or more of the following adjuvants: binders such as microcrystalline cellulose, gum tragacanth or gelatin; excipients such as starch or lactose, disintegrating agents such as alginic acid, Primogel, corn starch and the like; lubricants such as magnesium stearate or Sterotex; glidants such as colloidal silicon dioxide; and sweetening agents such as sucrose or saccharin may be added or a flavoring agent such as peppermint, methyl salicylate or orange flavoring. When the dosage unit form is a capsule, it may contain, in addition to materials of the above type, a liquid carrier such as polyethylene glycol or a fatty oil. Other dosage unit forms may contain other various materials that modify the physical form of the dosage unit, for example, as coatings. Thus, tablets or pills may be coated with sugar, shellac, or other coating agents. A syrup may contain, in addition to the present compounds, sucrose as a sweetening agent and certain preservatives, dyes and colorings and flavors. Materials used in preparing these various compositions should be pharmaceutically pure and non-toxic in the amounts used.

A formulation useful for the administration of R-(-)-N-methyl 3-((2-methylphenyl)oxy)-3-phenyl-1-aminopropane hydrochloride (atomoxetine) comprises a dry mixture of R-(-)-N-methyl 3-((2-methylphenyl)oxy)-3-phenyl-1-aminopropane hydrochloride with a diluent and lubricant. A starch, such as pregelatinized corn starch, is a suitable diluent and a silicone oil, such as dimethicone, a suitable lubricant for use in hard gelatin capsules. Suitable formulations are prepared containing about 0.4 to 26% R-(-)-N-methyl 3-((2-methylphen-yl)oxy)-3-phenyl-1-aminopropane hydrochloride, about 73 to 99% starch, and about 0.2 to 1.0% silicone oil. The following tables illustrate particularly preferred atomoxetine formulations:

| **Ingredient (%)** | **2.5 mg** | **5 mg** | **10 mg** | **18 mg** | **20 mg** | **25 mg** | **40 mg** | **60 mg** |
|---|---|---|---|---|---|---|---|---|
| R-(-)-N-methyl 3-((2-methylphenyl)oxy)-3-phenyl-1-aminopropane hydrochloride | 1.24 | 2.48 | 4.97 | 8.94 | 9.93 | 12.42 | 19.87 | 22.12 |
| Dimethicone | 0.5 | 0.5 | 0.5 | 0.5 | 0.5 | 0.5 | 0.5 | 0.5 |
| Pregelatinized Starch | 98.26 | 97.02 | 94.53 | 90.56 | 89.57 | 87.08 | 79.63 | 77.38 |

| **Ingredient (mg/capsule)** | **2.5 mg** | **5 mg** | **10 mg** | **18 mg** | **20 mg** | **25 mg** | **40 mg** | **60 mg** |
|---|---|---|---|---|---|---|---|---|
| R-(-)-N-methyl 3-((2-methylphenyl)oxy)-3-phenyl-1-aminopropane hydrochloride | 2.86 | 5.71 | 11.4 3 | 20.5 7 | 22.8 5 | 28.5 7 | 45.7 1 | 68.5 6 |
| Dimethicone | 1.15 | 1.15 | 1.15 | 1.15 | 1.15 | 1.15 | 1.15 | 1.55 |
| Pregelatinized Starch | 225.99 | 223.14 | 217.42 | 208.28 | 206.00 | 200.28 | 183.14 | 239.89 |
| **Capsule Fill Weight (mg)** | 230 | 230 | 230 | 230 | 230 | 230 | 230 | 310 |
| **Capsule Size** | 3 | 3 | 3 | 3 | 3 | 3 | 3 | 2 |

For the purpose of parenteral therapeutic administration, the compounds of the present invention may be incorporated into a solution or suspension. These preparations typically contain at least 0.1% of a compound of the invention, but may be varied to be between 0.1 and about 90% of the weight thereof. The amount of (R)-N-methyl-3-(2-methylthiophenoxy)-3-phenylpropylamine present in such compositions is such that a suitable dosage will be obtained. The solutions or suspensions may also include one or more of the following adjuvants: sterile diluents such as water for injection, saline solution, fixed oils, polyethylene glycols, glycerine, propylene glycol or other synthetic solvents; antibacterial agents such as benzyl alcohol or methyl paraben; antioxidants such as ascorbic acid or sodium bisulfite; chelating agents such as ethylene diaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. Preferred compositions and preparations may be determined by one skilled in the art.

### Inhibition or norepinephrine reuptake

The ability of compounds to inhibit the reuptake of norepinephrine may be measured by the general procedure of Wong, et al., *supra.*

Male Sprague-Dawley rats weighing 150-250 gm are decapitated and brains are immediately removed. Cerebral cortices are homogenized in 9 volumes of a medium containing 0.32 M sucrose and 10 mM glucose. Crude synaptosomal preparations are isolated after differential centrifugation at 1000 x g for 10 minutes and 17,000 x g for 28 minutes. The final pellets are suspended in the same medium and kept in ice until use within the same day.

Synaptosomal uptake of 3H-norepinephrine is determined as follows. Cortical synaptosomes (equvalent to 1 mg of protein) are incubated at 370C for 5 minutes in 1 mL Krebs-bicarbonate medium containing also 10 mM glucose, 0.1 mM iproniazide, 1 mM ascorbic acid, 0.17 mM EDTA and 50 nM ³H-norepinephrine. The reaction mixture is immediately diluted with 2 mL of ice-chilled Krebs-bicarbonate buffer and filtered under vacuum with a cell harvester (Brandel, Gaithersburg, MD). Filters are rinsed twice with approximately 5 mL of ice-chilled 0.9% saline and the uptake of ³H-norepinephrine assessed by liquid scintillation counting. Accumulation of ³H-norepinephrine at 4°C is considered to be background and is subtracted from all measurements. The concentration of the test compound required to inhibit 50% of the ³H-norepinephrine accumulation (IC₅₀ values) are determined by linear regression analysis.

The present invention provides the use of a selective norepinephrine reuptake inhibitor selected from the group consisting of atomoxetine and (R)-N-methyl-3-(2-methylthiophenoxy)-3-phenylpropylamine for the manufacture of a medicament for the treatment of cognitive failure. Cognitive failure may present in patients suffering from a number of disorders. The compounds of the present invention are useful for the treatment of cognitive failure associated with disorders classified in the Diagnostic and Statistical Manual of Mental Disorders, 4th Version, published by the American Psychiatric Association (DSM-IV). The DSM code numbers are supplied below for the convenience of the reader.

| | | |
|---|---|---|
| Delirium Due to a General Medical Condition | | 293.0 |
| Delirium Not Otherwise Specified | | 780.09 |

| Dementia of the Alzheimer's Type | | |
|---|---|---|
| | Early Onset with Delirium | 290.11 |
| | Early Onset with Delusions | 290.12 |
| | Early Onset Uncomplicated | 290.10 |
| | Late Onset with Delirium | 290.3 |
| | Late Onset with Delusions | 290.20 |
| | Late Onset Uncomplicated | 290.0 |
| | Uncomplicated | 290.40 |
| Dementia Due to HIV Disease | | 294.1 |
| Dementia Due to Head Trauma | | 294.1 |
| Dementia Due to Parkinson's Disease | | 294.1 |
| Dementia Due to Huntington's Disease | | 294.1 |
| Dementia Due to Pick's Disease | | 290.10 |
| Dementia Due to Creutzfeldt-Jakob Disease | | 290.10 |
| Dementia Due to Other General Medical Conditions | | 294.1 |
| Dementia Not Otherwise Specified | | 294.8 |
| Amnestic Disorder Due to a General Medical Condition | | 294.0 |
| Amnestic Disorder Not Otherwise Specified | | 294.8 |
| Cognitive Disorder Not Otherwise Specified | | 294.9 |
| Paranoid Type Schizophrenia | | 295.30 |
| Disorganized Type Schizophrenia | | 295.10 |
| Catatonic Type Schizophrenia | | 295.20 |
| Undifferentiated Type Schizophrenia | | 295.90 |
| Residual Type Schizophrenia | | 295.60 |
| Schizophreniform Disorder | | 295.40 |
| Schizoaffective Disorder | | 295.70 |

The skilled artisan will appreciate that the disorders listed above are illustrative of those indications where cognitive failure may appear, and is not intended to limit the scope of the present invention in any way.

Psychotic conditions to be treated by the adjunctive therapy aspect of the present invention include schizophrenia, schizophreniform diseases, acute mania, and schizoaffective disorders. The titles given these conditions represent multiple disease states. The following list illustrates a number of these disease states, many of which are classified in the Diagnostic and Statistical Manual of Mental Disorders, 4th Edition, published by the American Psychiatric Association (DSM). The DSM code numbers for these disease states are supplied below, when available, for the convenience of the reader.

| | |
|---|---|
| Paranoid Type Schizophrenia | 295.30 |
| Disorganized Type Schizophrenia | 295.10 |
| Catatonic Type Schizophrenia | 295.20 |
| Undifferentiated Type Schizophrenia | 295.90 |
| Residual Type Schizophrenia | 295.60 |
| Schizophreniform Disorder | 295.40 |
| Schizoaffective Disorder | 295.70 |

The present invention is also useful for the treatment of cognitive failure related to the onset of menopause.

The method of the present invention is effective in the treatment of patients who are children, adolescents or adults, and there is no significant difference in the symptoms or the details of the manner of treatment among patients of different ages. In general terms, however, for purposes of the present invention, a child is considered to be a patient below the age of puberty, an adolescent is considered to be a patient from the age of puberty up to about 18 years of age, and an adult is considered to be a patient of 18 years or older.

### EXAMPLE 1

The immediate-early gene, c-fos, and its protein products have been increasingly utilized as markers for neuronal activation (Dragunow and Faull, J. Neurosci. Methods, 29, 261-265 (1989); Morgan and Curran, Prog. In Brain Res., 86, 287-294 (1990); Robertson, et al., J. Pharmacol. Exp. Ther., 271, 1058-1066 (1994)). C-fos activation is measured as illustrated below for atomoxetine.

Two hours after administration of atomoxetine (3 mg/kg, i.p.), the rats were deeply anesthetized with sodium pentobarbital (60 mg/kg, i.p.) and transcardially perfused with 100 ml of phosphate buffered saline (PBS) followed by 100 ml of 4% paraformaldehyde in PBS. The brain was rapidly removed, postfixed for 90 min in 4% paraformaldehyde and then was transferred to 30% sucrose at 4° C until saturated. After quick freezing, serial 30 µm sections were cut and placed in PBS until processed for immunohistochemistry. In brief, sections were incubated in PBS containing blocking serum and 0.5% Triton-X 100 for 1 hour. Sections were then incubated with anti-Fos antibody (Santa Cruz Biotechnology, Inc.) at 4°C overnight. Visualization of the Fos-like immunoreactivity was performed with a Vectastain ABC Elite Kit (Vector Labs, Burlingame, CA) using the standard protocol supplied with the kit. Nickel-intensified diaminobenzidine (DAB) was used as the chromagen to yield a gray-black precipitation product. Following visualization of the Fos immunoreactivity, the sections were mounted on gelatin-coated glass slides and allowed to dry. The sections were then dehydrated and cover slipped. Fos expressing cells were quantitated using the MCID M2 imaging system (Imaging Research, St. Catherines, Ontario).

Surprisingly, atomoxetine increased the expression of c-fos only in the cortical areas as demonstrated by the data in the following table.

| Treatment | Prefrontal Cortex** | Nucleus Accumbens** | Striatum** |
|---|---|---|---|
| Vehicle | 80±28 | 129±33 | 118±26 |
| Atomoxetine | 296±26* | 152±44 | 102±35 |

| | | | |
|---|---|---|---|
| *=p<0.001 ** Fos positive cells/mm² | | | |

## Claims

1. Use of a selective norepinephrine reuptake inhibitor selected from atomoxetine and (R)-N-methyl-3-(2-methylthiophenoxy)-3-phenylpropylamine, or a pharmaceutically acceptable salt thereof, as sole active ingredient for the manufacture of a medicament for the treatment of cognitive failure.

2. Use according to claim 1, wherein said selective norepinephrine reuptake inhibitor is atomoxetine.

3. Use according to Claim 2, wherein said atomoxetine is in the form of a hydrochloride salt.

4. Use according to claim 1, wherein said selective norepinephrine reuptake inhibitor is (R)-N-methyl-3-(2-methylthiophenoxy)-3-phenylpropylamine.

5. Use according to Claim 4, wherein (R)-N-methyl-3-(2-methylthiophenoxy)-3-phenylpropylamine is in the form of a hydrochloride salt.

6. Use according to any one of Claims 1-5, wherein cognitive failure due to a delirium is treated.

7. Use according to any one of Claims 1-5, wherein cognitive failure due to schizophrenia is treated.

8. Use according to any one of Claims 1-5, wherein cognitive failure due to a dementia is treated.

9. Use according to claim 8, wherein said dementia is due to Alzheimer's disease.

10. Use according to claim 8, wherein said dementia is selected from the group consisting of Lewy body dementia, vascular dementia, and Binswanger's dementia.

11. Use according to claim 8, wherein said dementia appears in a patient with Parkinson's disease, progressive supranuclear palsy, Huntington's disease, Pick's disease, frontal lobe dementia syndromes, dementia pugilistica, normal-pressure hydrocephalus, subdural hematoma, Creutzfeldt-Jakob disease, Gerstmann-Straussler-Scheinker disease, general paresis, AIDS, or schizophrenia.

## Patentansprüche

1. Verwendung eines selektiven Norepinephrinwiederaufnahmeinhibitors, ausgewählt aus Atomoxetin und (R)-N-Methyl-3-(2-methylthiophenoxy)-3-phenylpropylamin oder einem pharmazeutisch akzeptablen Salz hiervon, als einzigem Wirkstoff zur Herstellung eines Arzneimittels für die Behandlung eines Kognitionsausfalls.

2. Verwendung nach Anspruch 1, worin der selektive Norepinephrinwiederaufnahmeinhibitor Atomoxetin ist.

3. Verwendung nach Anspruch 2, worin das Atomoxetin in der Form eines Hydrochloridsalzes vorliegt.

4. Verwendung nach Anspruch 1, worin der selektive Norepinephrinwiederaufnahmeinhibitor (R)-N-Methyl-3-(2-methylthiophenoxy)-3-phenylpropylamin ist.

5. Verwendung nach Anspruch 4, worin (R)-N-Methyl-3-(2-methylthiophenoxy)-3-phenylpropylamin in der Form eines Hydrochloridsalzes vorliegt.

6. Verwendung nach einem der Ansprüche 1 bis 5, worin ein Kognitionsausfall infolge eines Deliriums behandelt wird.

7. Verwendung nach einem der Ansprüche 1 bis 5, worin ein Kognitionsausfall infolge einer Schizophrenie behandelt wird.

8. Verwendung nach einem der Ansprüche 1 bis 5, worin ein Kognitionsausfall infolge einer Demenz behandelt wird.

9. Verwendung nach Anspruch 8, worin die Demenz eine Folge von Alzheimer-Krankheit ist.

10. Verwendung nach Anspruch 8, worin die Demenz aus der Gruppe ausgewählt ist, die besteht aus Lewy-Körperdemenz, Vaskulardemenz und Binswanger-Demenz.

11. Verwendung nach Anspruch 1, worin die Demenz vorkommt bei einem Patienten mit Parkinson-Krankheit, progressiver supranuklearer Palsie, Huntington-Krankheit, Pick-Krankheit, Stirnlappendemenzsyndrom, Dementia pugilistica, Normaldruckhydrozephalus, Subduralhämatom, Creutzfeld-Jakob-Krankheit, Gerstmann-Straussler-Scheinker-Krankheit, allgemeiner Parese, AIDS oder Schizophrenie.

## Revendications

1. Utilisation d'un inhibiteur sélectif de la réabsorption de la noradrénaline choisi parmi l'atomoxetine et la (R)-N-méthyl-3-(2-méthylthiophénoxy)-3-phénylpropylamine, ou un sel pharmaceutiquement acceptable de ceux-ci, comme unique ingrédient actif pour la préparation d'un médicament destiné au traitement du déficit cognitif.

2. Utilisation selon la revendication 1, dans laquelle ledit inhibiteur sélectif de la réabsorption de la noradrénaline est l'atomoxetine.

3. Utilisation selon la revendication 2, dans laquelle ledit atomoxetine est sous forme d'un sel chlorhydrate.

4. Utilisation selon la revendication 1, dans laquelle ledit inhibiteur sélectif de la réabsorption de la noradrénaline est la (R)-N-méthyl-3-(2-méthylthiophénoxy)-3-phénylpropylamine.

5. Utilisation selon la revendication 4, dans laquelle la (R)-N-méthyl-3-(2-méthylthiophénoxy)-3-phénylpropylamine est sous forme d'un sel chlorhydrate.

6. Utilisation selon l'une quelconque des revendications 1-5, dans laquelle on traite le déficit cognitif dû au delirium.

7. Utilisation selon l'une quelconque des revendications 1-5, dans laquelle on traite le déficit cognitif dû à la schizophrénie.

8. Utilisation selon l'une quelconque des revendications 1-5, dans laquelle on traite le déficit cognitif dû à la démence.

9. Utilisation selon la revendication 8, dans laquelle ladite démence est due à la maladie d'Alzheimer.

10. Utilisation selon la revendication 8, dans laquelle ladite démence est choisie dans le groupe constitué par la démence à corps de Lewy, la démence vasculaire et la démence de Binswanger.

11. Utilisation selon la revendication 8, dans laquelle ladite démence survient chez un patient souffrant de la maladie de Parkinson, de la paralysie supranucléaire progressive, de la maladie de Huntington, de la maladie de Pick, de syndromes de démence du lobe frontal, de la démence pugilistique, de l'hydrocéphale à pression normale, d'un hématome subdural, de la maladie de Creutzfeldt-Jakob, de la maladie de Gerstmann-Straussler-Scheinker, d'une parésie générale, du SIDA ou de la schizophrénie.
